(19) Europäisches Patentamt
European Patent Office
Office européen des brevets

(11) **EP 4 765 037 A1**

(12) **EUROPEAN PATENT APPLICATION**
published in accordance with Art. 153(4) EPC

(43) Date of publication:
**24.06.2026 Bulletin 2026/26**

(21) Application number: **24853415.8**

(22) Date of filing: **04.07.2024**

(51) International Patent Classification (IPC):
**G06V 10/764** (2022.01)

(52) Cooperative Patent Classification (CPC):
Y02A 90/10

(86) International application number:
**PCT/CN2024/103559**

(87) International publication number:
**WO 2025/036000 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.08.2023 CN 202311040222**

(71) Applicant: **Tianjin Yujin Artificial Intelligence Medical Technology Co., Ltd
Tianjin 300457 (CN)**

(72) Inventors:
• **WANG, Yufeng**
  **Tianjin 300457 (CN)**
• **MU, Jinbao**
  **Tianjin 300457 (CN)**

(74) Representative: **Cabinet Chaillot
16/20, avenue de l'Agent Sarre
B.P. 74
92703 Colombes Cedex (FR)**

(54) **LESION FEATURE RECOGNITION SYSTEM FOR GASTROINTESTINAL ENDOSCOPY**

(57)    Disclosed in the present invention is a lesion feature recognition system for gastrointestinal endoscopy. By utilizing the system, the accuracy and efficiency of lesion feature recognition under gastrointestinal endoscopy can be improved, and the shortcomings including limited recognition capability, poor anti-interference performance, high demand for computing power, high misdiagnosis rate and the like under conventional AI-assisted endoscope inspection technology are overcome.

FIG.1

**Description**

CROSS-REFERENCE TO RELATED APPLICATION

The present application claims the priority of Chinese Patent Application No. 202311040222.1, originally filed with the Chinese Patent Office on August 17, 2023 and entitled "System for Recognizing Gastrointestinal Endoscopic Lesion Features", which is incorporated in its entirety herein by reference.

TECHNICAL FIELD

The present invention relates to the field of artificial intelligence, in particular to a system for recognizing gastrointestinal endoscopic lesion features.

DESCRIPTION OF RELATED ART

The application of artificial intelligence (AI) in clinical medicine has become a hot research topic. A primary approach involves constructing a multi-layered deep learning model, collecting a large number of gastrointestinal endoscopic lesion images from hospitals, requiring endoscopists to annotate lesions represented in the images as training data, and allowing the AI to learn data features, to achieve the purpose of identifying some gastrointestinal diseases ultimately. Typically, it provides real-time assistance for an endoscopist during examinations through software prompts. When lesions are detected, a prompt signal appears on a screen to remind the endoscopist to perform observation operations, and to provide a preliminary diagnostic result. Such technologies are being researched and applied across multiple areas including esophageal cancer, gastric cancer, and colorectal adenomatous polyps. They have demonstrated clinical value by enhancing the examination efficiency of endoscopists and reducing missed diagnoses. Beyond localized lesions, AI-assisted diagnosis research has also made continuous progress in lesions such as chronic atrophic gastritis and ulcerative colitis. Although the AI has already achieved medical-level proficiency in recognizing some static images of lesions, and even some technologies can diagnose the malignant or benign risk, in general, such applications still fall short of the maturity required for clinical use. With a high false positive rate, they remain distant from widespread adoption, with most achievements currently confined to scientific research and clinical trials.

At present, the research focus of mainstream AI technologies is primarily on recognizing and diagnosing endoscopic features for a single disease, and such technologies do not have the function of differentiating multiple diseases. To add diagnostic capabilities for a new disease, a new model must be built. After completing new sample training, the model operates in parallel with an original model to enable diagnosis. Clinically, dozens of diseases are commonly diagnosed through digestive endoscopy. To effectively assist endoscopists, the AI technologies must possess the diagnosing and identifying capabilities to simultaneously recognize dozens of diseases. This places higher demands on both computing power and accuracy for AI technologies utilizing single-disease models. It is challenging for the current hardware capabilities of small computers to simultaneously satisfy the requirements of low latency and versatility. Second, the AI technologies require a large number of samples as a prerequisite for model training. With the addition of diagnosis requirements for a new disease, corresponding costs for sample collection and annotation also increase. This also prevents such AI applications from rapidly expanding their diagnostic capabilities. Third, current technical solutions exhibit significant limitations in reaching diagnostic conclusions for diseases characterized by complex and variable features. Due to technical limitations, the more variable and complex the features of a single lesion are, the more challenging it becomes for the AI to reduce false positives and false negatives to an acceptable level.

In the field of endoscopic diagnosis of gastrointestinal diseases, various AI technologies and methods currently exist, such as deep learning, convolutional neural networks, support vector machines, and Bayesian networks. These technologies and methods can theoretically be used for endoscopic diagnosis of gastrointestinal diseases. However, they have limitations or insurmountable difficulties in achieving the goal of identifying various diseases. For example, 1) recognition of a single disease or lesion: some AI technologies may only be capable of recognizing a single disease or lesion, rather than simultaneously recognizing several different lesions and diseases. 2) High sample requirements: some AI technologies require an extensive amount of labeled data and the expertise and experience of professional doctors in order to train high-quality models. 3) High model complexity: For some AI technologies, building a plurality of models or retraining models may increase model complexity and training time. 4) High misdiagnosis rate: Some AI technologies may fail to account for the interactions and similarities between different lesions, potentially leading to misdiagnosis issues. Consequently, to address the aforementioned technical problems, a novel solution is required. Only then can AI-assisted digestive endoscopy diagnosis technologies become widely applicable.

BRIEF SUMMARY OF THE INVENTION

Technical problem to be solved:

Traditional AI models are usually trained and optimized for a single disease or a single lesion, requiring an extensive amount of labeled data and expertise and experience of professional doctors. Moreover, in practical application, interactions and similarities may exist between different lesions, and a traditional single-disease training method cannot deal with these problems well. In addition, establishing a model for each disease separately increases complexity of the models, cost of sample processing, and training time, limiting efficiency and feasibility in practical applications.

In particular, an aspect of the present invention aims to solve the problems existing in the traditional AI models when they assist in endoscopic diagnosis of gastrointestinal diseases, and proposes a novel system. That is, features of different lesion types are analyzed. A plurality of feature points consistent with clinical diagnosis bases are extracted, and classified and summarized as a recognition target of an AI model. In this way, different lesions and diseases can be automatically recognized simultaneously, to avoid issues of establishing a plurality of models and retraining. Correct disease diagnosis can be obtained by performing secondary determination of a recognized feature combination. For clinical work where patient conditions are unpredictable and dozens of lesions require recognition, this method can enhance diagnostic accuracy and efficiency, providing endoscopists with a truly usable auxiliary diagnostic tool, and make up for shortcomings of single diagnostic capabilities, poor anti-interference, high computing power requirements, and a high misdiagnosis rate of a traditional AI-assisted endoscopic examination technology.

Technical solution:

A system for recognizing gastrointestinal endoscopic lesion features includes:

1) an image collection module;
2) a gastrointestinal endoscopic multi-level feature recognition and training module including a first multi-task classification neural network configured to recognize n preset different categories of gastrointestinal endoscopic feature groups and label values in the gastrointestinal endoscopic feature groups, where $2 \leq n \leq 10$; and
3) a feature result collection module configured to collect recognition results output by the gastrointestinal endoscopic multi-level feature recognition and training module,

where the gastrointestinal endoscopic feature groups comprise a local lesion feature group, and a label value of the local lesion feature group includes a local depressed lesion, a local elevated lesion, or a local flat lesion; and a downstream network of the local lesion feature group includes a local lesion multi-level feature recognition and training module, the local lesion multi-level feature recognition and training module includes a second multi-task classification neural network configured to recognize m preset different categories of local lesion features and complementary identification values corresponding to the categories, and $1 \leq m \leq 5$.

According to the other aspect of the present invention, an electronic apparatus is provided. The electronic apparatus includes an electronic apparatus body and the above system for recognizing gastrointestinal endoscopic lesion features. The system for recognizing gastrointestinal endoscopic lesion features is mounted on the electronic apparatus body.

The present invention has the beneficial effects as follows:
According to the present invention, a novel AI model based on feature point classification and summarization is adopted. A plurality of different lesions and diseases can be recognized simultaneously. The issues of establishing a plurality of models and retraining are avoided. The accuracy and efficiency of endoscopic diagnosis of gastrointestinal diseases are improved. Specifically:

1) The accuracy of diagnosis is improved: the AI model adopted by the present invention can automatically recognize, classify, and summarize a plurality of different lesions and diseases simultaneously, avoiding the issues of establishing a plurality of models and retraining. Through the secondary determination of the recognized feature combination, more accurate disease diagnosis results can be obtained, the misdiagnosis rate can be reduced, and the accuracy of diagnosis can be improved.

2) The efficiency of diagnosis is improved: the traditional AI models need to establish a model for each disease separately, increasing the complexity of the models, the cost of sample processing, and the training time, and limiting the efficiency and the feasibility of the AI models in practical applications. The AI model based on feature point classification and summarization adopted by the present invention can recognize a plurality of different lesions and diseases simultaneously. The issues of establishing a plurality of models and retraining are avoided. The efficiency of endoscopic diagnosis of gastrointestinal diseases is improved.

3) A workload of manual labeling is reduced: the AI model adopted by the present invention can automatically analyze, classify, and summarize features of different lesions and diseases, reducing the workload of manual labeling.

4) A clinical application value is improved: the AI model of the present invention can recognize a plurality of different lesions and diseases simultaneously, providing endoscopists with a truly usable auxiliary diagnostic tool. examination efficiency of the endoscopists can be improved. Missed diagnosis can be reduced. A value of AI in clinical application can be expanded. The AI technology application can be widely popularized.

BRIEF DESCRIPTION OF DRAWINGS

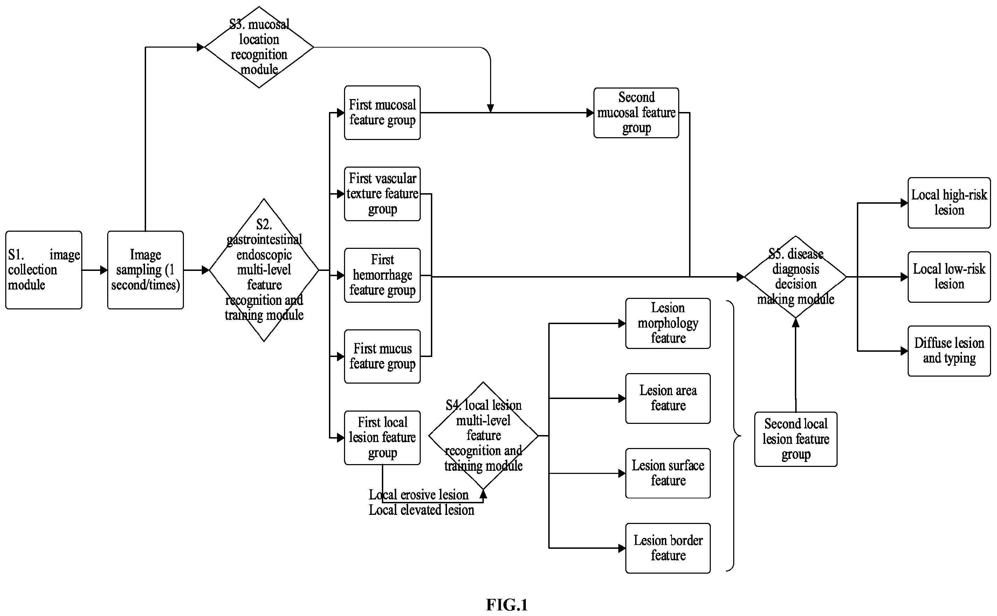

FIG. 1 is a schematic diagram of a system for recognizing gastrointestinal endoscopic lesion features constructed in an example of the present invention.

DETAILED DESCRIPTION OF THE EMBODIMENTS

A system for recognizing gastrointestinal endoscopic lesion features is disclosed in the present invention. Those skilled in the art can refer to contents herein to appropriately improve process parameters for implementation. It is specifically noted that all similar substitutions and modifications will be apparent to those skilled in the art and are deemed to be included in the present invention. It will be apparent to those skilled in the art that modifications or suitable variations and combinations of what is described herein can be made to practice and apply the disclosed technology without departing from the content, spirit and scope of the present disclosure.

In the present invention, scientific and technical terms used herein have the meanings commonly understood by those skilled in the art, unless otherwise specified. Throughout the specification and claims, unless explicitly indicated otherwise, the term "comprise," or its variations of "include" or "contain," and the like, will be understood to include stated elements or components but not to exclude other elements or components. The terms "such as," "for example," and the like are intended to refer to illustrative embodiments and are not intended to limit the scope of the present invention.

In order to enable those skilled in the art to better understand the technical solutions of the present invention, the present invention is described in further detail below in combination with specific examples.

As shown in FIG. 1, disclosed in the example is a system for recognizing gastrointestinal endoscopic lesion features of the present invention. The system includes:

S1. image collection module.

The image collection module is configured to obtain images stored in real time or in advance in a medium. The image collection module may include a video obtaining module and/or a picture obtaining module. In some embodiments of the present invention, the image collection module may use a video collection card to transmit high-definition multimedia interface (HDMI), digital video interactive (DVI), standard data interface (SDI), S-Video, and other digital/analog video signals to a computer, read the video signals through opencv, and transcode the video signals into images in RGB image format frame by frame.

In some embodiments of the present invention, when the image collection module obtains a video real-time image signal output by an endoscope apparatus, the image collection module performs interval sampling on the obtained frame-by-frame images, for example, with an interval sampling rate of 1 second/time, to obtain images to be recognized, and records frame numbers of the images to be recognized.

S2. gastrointestinal endoscopic multi-level feature recognition and training module.

The gastrointestinal endoscopic multi-level feature recognition and training module includes a first multi-task classification neural network configured to recognize n preset different categories of gastrointestinal endoscopic feature groups and label values in the gastrointestinal endoscopic feature groups, where $2 \leq n \leq 10$. The gastrointestinal endoscopic feature groups include a local lesion feature group. In some embodiments of the present invention, n may be 2, 3, 4, 5, 6, 7, 8, 9, or 10. In an embodiment of the present invention, n=5. That is, the gastrointestinal endoscopic feature groups include five different categories, that is, a mucosal feature group, a vascular texture feature group, a hemorrhage feature group, a mucus feature group, and the local lesion feature group. In some other embodiments of the present invention, at least one of the mucosal feature group, the vascular texture feature group, the hemorrhage feature group, or the mucus feature group, or other relevant gastrointestinal endoscopic feature groups may be selected. Mucosal features, vascular texture features, hemorrhage features, and mucus features mentioned above belong to diffuse lesion features.

In some embodiments of the present invention, label values corresponding to the above different categories of gastrointestinal endoscopic feature groups may include that, for example, label values of the mucosal feature group include mucosal thinning, a linear erythematous mucosa, a patchy erythematous mucosa, a mucosal edema, an island-like mucosa, a residual glandular mucosa, a gastric mucosa, an intestinal mucosa, or an intestinal metaplasia; label values of the vascular texture feature group include obscured submucosal vessels, linear vessels, dendritic vessels, or spotty and patchy vessels; label values of the hemorrhage feature group include no hemorrhage, spotty and patchy hemorrhage, mild hemorrhage, or massive hemorrhage; label values of the mucus feature group include yellowish mucus, viscous mucus, bile reflux mucus, clear mucus, or a mucus lake; and a label value of the local lesion feature group includes a local depressed lesion, a local elevated lesion, or a local flat lesion.

To achieve an objective of the present invention, in some embodiments of the present invention, the first multi-task classification neural network includes one main feature extraction network configured to recognize the n preset different categories of gastrointestinal endoscopic feature groups, and n branch feature extraction networks located downstream of the main feature extraction network and configured to recognize the label values in the gastrointestinal endoscopic feature groups respectively. In an embodiment of the present invention, n=5. A method for constructing the first multi-task classification neural network includes:

Step A) at least three (for example, 4, 6, 8, 9, and 10) multi-branch parallel feature extraction units are used to form the main feature extraction network, and a first main feature extraction network feature map is obtained through operation.
Step B) at least three (for example, 4, 5, 6, and 8) multi-branch parallel feature extraction units are used to form an n-th branch feature extraction network; and the first main feature extraction network feature map is input, and finally an n-th branch feature extraction network feature map is obtained through multi-branch parallel convolution operation.

A method for designing a multi-branch parallel convolution operation unit includes:

a) a slicing unit is constructed and configured to divide an original input feature map into four groups to obtain a first slice feature group, a second slice feature group, a third slice feature group, and a fourth slice feature group.
b) one grouping feature extraction unit is constructed by three depthwise separable convolutions, and features are extracted from the first slice feature group, the second slice feature group, and the third slice feature group separately. The three depthwise separable convolutions have sizes of 3*3, 1 * 13, and 13* 1 respectively, and are used for extracting spatial features, vertical channel features, and horizontal channel features. Finally, an extracted spatial feature map, a vertical channel feature map, a horizontal channel feature map, and the fourth slice feature group are re-concatenated, and a first output is obtained through one regularization layer.
c) a feature enhancement unit is constructed, where the feature enhancement unit is composed of one 1*1 convolution layer, an activation function layer, one 1 * 1 convolution layer, and one residual connection, a second output is obtained by performing series operation on a 1*1 convolution, an activation function, and an 1*1 convolution, and a third output is obtained by performing horizontal direction feature map concatenating on the original input feature map and the second output through the residual connection, that is, an output result of the multi-branch parallel convolution operation unit is obtained.

The 1*1 convolution layer is formed by conventional technical means in the art, and the activation function is GeLU.
Step C) a first prediction layer, a second prediction layer, a third prediction layer, a fourth prediction layer, and a fifth prediction layer are constructed by using a fully connected layer and a Sigmoid function as a combination unit. Specifically, a first branch feature map, a second branch feature map, a third branch feature map, a fourth branch feature map, and a fifth branch feature map are input to the fully connected layer, the network layer compresses the high-dimensional feature maps into a 1-dimensional vector, and finally label recognition results of a first feature group, a second feature group, a third feature group, a fourth feature group, and a fifth feature group are obtained through Sigmoid function operation. A Sigmoid operation formula is:

$$p_k = Sigmoid(W_k^T \cdot F + b_k)$$

where $p_k$ indicates a probability of solving for category k, $W_k^T$ is a trainable hyperparameter for category k, and $b_k$ is a trainable biased hyperparameter for category k.
In some embodiments of the present invention, training sets are input to the first multi-task classification neural network in batches for training.
A source of the training sets is: gastrointestinal picture data is collected according to a plurality of groups of preset multi-label feature results, feature labels are labeled one by one to obtain a first feature label data set, and the first feature label data set is split into a training set and a verification set according to a ratio of 8:2. The feature groups and the corresponding label values are shown in Table 1.

Table 1

| Feature group name | Feature label value |
|---|---|
| **Mucosal feature group** | Mucosal thinning, a linear erythematous mucosa, a patchy erythematous mucosa, a mucosal edema, an island-like mucosa, a residual glandular mucosa, a gastric mucosa, an intestinal mucosa, and an intestinal metaplasia |
| **Vascular texture feature group** | Obscured submucosal vessels, linear vessels, dendritic vessels, and spotty and patchy vessels |
| **Hemorrhage feature group** | No hemorrhage, spotty and patchy hemorrhage, mild hemorrhage, and massive hemorrhage |
| **Mucus feature group** | Yellowish mucus, viscous mucus, bile reflux mucus, clear mucus, and a mucus lake |
| **Lesion feature group** | Local erosive lesion, local elevated lesion, diffuse lesion |

In order to achieve an objective of the present invention, a method for training the first multi-task classification neural network includes:

Step D) an original label value output correspondingly by each of the gastrointestinal endoscopic feature groups is predicted, calculation is performed through a BCE cross-entropy loss function, an n-th loss function is obtained, and a total loss of the network is calculated in a weighted summation manner, where a specific formula is as follows:

$$L_k = -\frac{1}{N}\sum_{i=1}^{N}[y_i \, log\,(p_i) + (1 - y_i)\, log\,(1 - p_i)]$$

where k is a number of branches of the feature groups; $L_k$ is a derivative of a loss for k feature branches; N is a number of pictures in a training batch; i is a corresponding category; $y_i$ is a true label value of category i in the pictures, that is, $y_i$ is 1 in a case where a picture belongs to category i, and $y_i$ is 0 in a case where a picture does not belong to category i; and $p_i$ is a prediction confidence coefficient of the picture for category i;

$$L = w_1 \times L_1 + w_2 \times L_2 + \ ...... + w_k \times L_k$$

where L is the total loss, and w is a weight of the loss function; and the total loss is obtained by summing.

In the present invention, an adjustment strategy for the weight of the loss function is stage attenuation. That is, fixed weights are [2.5, 2.5, 2.5, 2.5, 2.5] at the beginning of training, and are adjusted every 10 rounds. An adjustment basis is: in a case where $L_t < L_{(t-10)}$, $w_t = w_t * \theta$; and in a case where $L_t > L_{(t-10)}$, $w_t = w_t * (2 - \theta)$. Further, θ is an attenuation coefficient, and θ=0.85.

Step E) parameters of a neural network model are updated by using a backpropagation technology according to the total loss of the network, training is stopped in a case where a final loss function is stabilized to a preset value or reaches a preset maximum iteration number, and the first multi-task classification neural network trained in advance is obtained.

The first multi-task classification neural network constructed and trained is used for recognizing and predicting the gastrointestinal endoscopic lesion features. 1) The frame-by-frame images obtained by the image collection module are sampled at intervals with a sampling rate of 1 second/time, to obtain images to be recognized, and frame numbers of the images to be recognized are recorded. 2) Multi-group multi-label feature recognition is performed on the image to be recognized by using the first multi-task classification neural network constructed in advance to obtain a recognition result of a first mucosal feature group, a recognition result of a first vascular texture feature group, a recognition result of a first hemorrhage feature group, a recognition result of a first mucus feature group, and a recognition result of first local lesion feature group.

The recognition result of the first mucosal feature group, the recognition result of the first vascular texture feature group, the recognition result of the first hemorrhage feature group, the recognition result of the first mucus feature group, and the recognition result of the first local lesion feature group obtained above are input to a feature result collection module for appropriate purposes, which includes, but is not limited to, auxiliary diagnosis of gastrointestinal diseases.

To achieve an objective of the present invention, in some embodiments of the present invention, a downstream network of the local lesion feature group may further include a local lesion multi-level feature recognition and training module. The local lesion multi-level feature recognition and training module includes a second multi-task classification neural network configured to recognize m preset different categories of local lesion features and complementary identification values corresponding to the categories, and $1 \leq m \leq 5$. In some embodiments of the present invention, m may be 1, 2, 3, 4, or 5. In an embodiment of the present invention, m=4. That is, the local lesion features include four different categories, for example, a lesion morphology feature, a lesion area feature, a lesion surface feature, or a lesion border feature.

Further, S4. local lesion multi-level feature recognition and training module.

The local lesion multi-level feature recognition and training module includes a second multi-task classification neural network configured to recognize four preset different categories of local lesion features and complementary identification values corresponding to the categories. The second multi-task classification neural network may be obtained by using the same methods for constructing and training the first multi-task classification neural network as described above. In some embodiments of the present invention, a method for constructing the second multi-task classification neural network includes:

Step F) a lesion segmentation artificial intelligence neural network is constructed based on a DeepLabV3+ algorithm, and predicting images of the local lesion feature group, to obtain a first lesion segmentation map. Specifically,

i) Required data sets are constructed. A contour of a lesion is labeled. Targets include an elevated lesion target and an erosive lesion target. A first segmented lesion data set is obtained.

Second, multi-level labels of the lesion are labeled to construct a second label feature data set.

ii) A lesion segmentation model is trained according to the first segmented lesion data set to obtain a first lesion segmentation model. Input data is an image, and an output is a mask map containing only a segmented region.

Step G) a second multi-task classification neural network algorithm is use for predicting multi-level feature labels of the lesion. The first lesion segmentation map is input to obtain prediction results of a first lesion morphology feature group, a first lesion area feature group, a first lesion surface feature group, and a first lesion border feature group. Specifically,

i) The network is constructed by using the same method for constructing the first multi-task classification neural network as described above. Since only four feature groups are provided, four branches are set, and therefore subsequent four loss functions are provided.

ii) The second multi-task classification neural network algorithm constructed in advance is obtained through training, such that the second multi-task classification neural network algorithm has a lesion feature group label recognition capability. That is,

when recognition labels in a first lesion feature recognition group include "local erosion lesion" and "local elevated lesion," the second multi-task classification neural network algorithm constructed in advance is used for recognizing complementary identification values of the feature groups. A first complementary lesion morphology feature group, a first complementary lesion area feature group, a first complementary lesion surface feature group, and a first complementary lesion border feature group are obtained.

1) In the first complementary lesion morphology feature group, corresponding labels of "local erosive lesion" include: red and swollen, and not red and swollen; and corresponding labels of "local elevated lesion" include: flat, lateral, pedunculated, and irregular.

2) In the first complementary lesion area feature group, corresponding labels of "local erosive lesion" include: punctate, superficial and small, and deep and large; and corresponding labels of "local elevated lesion" include: multiple and solitary.

3) In the first complementary lesion surface feature group, corresponding labels of "local erosive lesion" include: no white coating, grayish white, yellowish white; and corresponding labels of "local elevated lesion" include: light white, light pink, and deep red.

4) In the first complementary lesion border feature group, corresponding labels of "local erosive lesion" include: smooth and burr; and corresponding labels of "local elevated lesion" include: smooth and rough.

Step H) a label conversion module is constructed, and the complementary identification values obtained in step G) are fused with the label values of the local lesion feature group, to obtain a label value of a new local lesion feature group (second local lesion feature group).

A recognition result of the second local lesion feature group, together with the recognition result of the first mucosal feature group, the recognition result of the first vascular texture feature group, the recognition result of the first hemorrhage feature group, and the recognition result of the first mucus feature group, may be input to the feature result collection module for appropriate purposes, which includes, but is not limited to, auxiliary diagnosis of gastrointestinal diseases.

To achieve an objective of the present invention, in some embodiments of the present invention, the system for recognizing gastrointestinal endoscopic lesion features further includes a mucosal location recognition module configured to convert the label values in the mucosal feature group according to a mucosal location and output the recognition result to the feature result collection module. The location includes esophagus, gastric body, gastric fundus, gastric antrum, duodenum, cecum, ascending colon, transverse colon, descending colon, sigmoid colon, or rectum.

Further, S3. mucosal location recognition module.

A method for constructing the mucosal location recognition module includes:

step I) a time-series segment classification model is used for classifying and recognizing a location of a site in a current video/picture to obtain a location recognition result.

i) Video clips of any appropriate duration during digestive endoscopy are collected.

ii) Data is preprocessed, dimensionality is reduced and an image is scaled to an appropriate size by using an interval sampling technology according to a total number of frames in a collection duration.

iii) ResNet-3D is used for inputting and training the preprocessed data, and parameters are updated by a backpropagation technology in the process, to obtain a preset time-series segment classification model, such that the model has a position recognition capability.

step J) result conversion is performed on the first mucosal feature group in a feature recognition result of a first mucosal feature group image to be recognized after sampling and obtained by the gastrointestinal endoscopic multi-level feature recognition and training module. Specifically,

i) Images read frame by frame are accumulated to obtain a segment set reaching an appropriate duration, and the segment set is processed by using the preprocessing module.

ii) Location prediction is performed by using the preset time-series segment classification model to obtain a frame number range and a prediction result corresponding to the location.

iii) The first mucosal feature group corresponding to a frame number in the interval is reversely searched for according to the frame number range. In a case where corresponding locations of the interval of the frame number range are "gastric body," "gastric fundus," and "gastric antrum", and the first feature group in the interval contains a label of "intestinal mucosa", the label of "intestinal mucosa" is removed from the first mucosal feature group, and a label of "intestinal metaplasia" is added.

iv) The label is added to the first mucosal feature group according to a recognition result corresponding to the location in the frame segment.

The result output by the mucosal location recognition module is a recognition result of the second mucosal feature group.

The recognition result of the second mucosal feature group, together with the recognition result of the first vascular texture feature group, the recognition result of the first hemorrhage feature group, the recognition result of the first mucus feature group, and the recognition result of the first local lesion feature group, may be input to the feature result collection module for appropriate purposes, which includes, but is not limited to, auxiliary diagnosis of gastrointestinal diseases.

In some other embodiments of the present invention, the recognition results output by the gastrointestinal endoscopic multi-level feature recognition and training module may include: the recognition result of the first/second mucosal feature group, the recognition result of the first vascular texture feature group, the recognition result of the first hemorrhage feature group, the recognition result of the first mucus feature group, and the recognition result of the first/second local lesion feature group. The recognition results are input to the feature result collection module for appropriate purposes, which includes, but is not limited to, auxiliary diagnosis of gastrointestinal diseases.

To achieve an objective of the present invention, in some embodiments of the present invention, the system for recognizing gastrointestinal endoscopic lesion features further includes a disease diagnosis decision making module configured to obtain a disease diagnosis result by using the recognition results.

Further, S5. disease diagnosis decision making module.

A method for constructing the disease diagnosis decision making module includes:

Step K) disease diagnosis labeling is performed on medical images in the first label data set, to obtain a first disease data set. Inputs of the data set are, for example, the recognition result of the second mucosal feature group, the recognition result of the first vascular texture feature group, the recognition result of the first hemorrhage feature group, the recognition result of the first mucus feature group, and the recognition result of the second local lesion feature group (that is, label values). An output is a disease category. In some embodiments of the present invention, the disease category includes, but is not limited to, ulcerative colitis and atrophic gastritis. In some other embodiments of the present invention, the output may alternatively be nature of a disease, including, for example, but not limited to, a local high-risk lesion, a local low-risk lesion, and a diffuse lesion and typing.

Step L) a preset disease diagnosis decision making algorithm is constructed. Specifically, a disease contained in the medical images is labeled based on an XGBOOST algorithm, and data is trained iteratively batch by batch to obtain the disease diagnosis decision making algorithm constructed in advance.

Step M) the disease diagnosis decision making module may further include a preprocessing module configured to align the recognition result of the second mucosal feature group, the recognition result of the first vascular texture feature group, the recognition result of the first hemorrhage feature group, the recognition result of the first mucus feature group, and the recognition result of the second local lesion feature group (that is, label values) to obtain an aggregate feature array.

Step N) the disease diagnosis decision making algorithm constructed in advance is used for performing inputting and algorithm inference on a feature set, to obtain the disease diagnosis result.

The above descriptions are merely preferred embodiments of the present invention. It should be noted that those of ordinary skill in the art may further make several improvements and modifications without departing from the principle of the present invention, but such improvements and modifications should be deemed as falling within the scope of protection of the present invention.

**Claims**

1.  A system for recognizing gastrointestinal endoscopic lesion features, comprising:

    1) an image collection module;
    2) a gastrointestinal endoscopic multi-level feature recognition and training module comprising a first multi-task classification neural network configured to recognize n preset different categories of gastrointestinal endoscopic feature groups and label values in the gastrointestinal endoscopic feature groups, wherein $1 \leq n \leq 10$; and
    3) a feature result collection module configured to collect recognition results output by the gastrointestinal endoscopic multi-level feature recognition and training module,
    wherein the gastrointestinal endoscopic feature groups comprise a local lesion feature group, and a label value of the local lesion feature group comprises a local depressed lesion, a local elevated lesion, or a local flat lesion; and a downstream network of the local lesion feature group comprises a local lesion multi-level feature recognition and training module, the local lesion multi-level feature recognition and training module comprises a second multi-task classification neural network configured to recognize m preset different categories of local lesion features and complementary identification values corresponding to the categories, and $1 \leq m \leq 5$.

2.  The system for recognizing gastrointestinal endoscopic lesion features according to claim 1, **characterized in that** the image collection module comprises a video obtaining module and/or a picture obtaining module,

    wherein the video obtaining module is configured to read a video signal output by an endoscope apparatus, to obtain video frame images; and
    preferably, the video obtaining module performs interval sampling on the obtained frame-by-frame images to obtain images to be recognized, and records frame numbers of the images to be recognized.

3.  The system for recognizing gastrointestinal endoscopic lesion features according to claim 1, **characterized in that** the gastrointestinal endoscopic feature groups further comprise at least one of a mucosal feature group, a vascular texture feature group, a hemorrhage feature group, or a mucus feature group; and
    preferably, label values of the mucosal feature group comprise mucosal thinning, a linear erythematous mucosa, a patchy erythematous mucosa, a mucosal edema, an island-like mucosa, a residual glandular mucosa, a gastric mucosa, an intestinal mucosa, or an intestinal metaplasia; label values of the vascular texture feature group comprise obscured submucosal vessels, linear vessels, dendritic vessels, or spotty and patchy vessels; label values of the hemorrhage feature group comprise no hemorrhage, spotty and patchy hemorrhage, mild hemorrhage, or massive hemorrhage; and label values of the mucus feature group comprise yellowish mucus, viscous mucus, bile reflux mucus, clear mucus, or a mucus lake.

4.  The system for recognizing gastrointestinal endoscopic lesion features according to claim 1, **characterized in that** the first multi-task classification neural network comprises one main feature extraction network configured to recognize the n preset different categories of gastrointestinal endoscopic feature groups, and n branch feature extraction networks located downstream of the main feature extraction network and configured to recognize the label values in the gastrointestinal endoscopic feature groups respectively, and a method for constructing the first multi-task classification neural network comprises:

step A) using at least three multi-branch parallel feature extraction units to form the main feature extraction network, and obtaining a first main feature extraction network feature map through operation;

step B) using at least three multi-branch parallel feature extraction units to form an n-th branch feature extraction network respectively; and inputting the first main feature extraction network feature map, and finally obtaining an n-th branch feature extraction network feature map through multi-branch parallel convolution operation, wherein a method for designing a multi-branch parallel convolution operation unit comprises:

a) constructing a slicing unit configured to divide an original input feature map into four groups to obtain a first slice feature group, a second slice feature group, a third slice feature group, and a fourth slice feature group;

b) constructing one grouping feature extraction unit by three depthwise separable convolutions, extracting features from the first slice feature group, the second slice feature group, and the third slice feature group separately, wherein the three depthwise separable convolutions have sizes of 3*3, 1*13, and 13*1 respectively, and are used for extracting spatial features, vertical channel features, and horizontal channel features, finally, re-concatenating an extracted spatial feature map, a vertical channel feature map, a horizontal channel feature map, and the fourth slice feature group, and obtaining a first output through one regularization layer; and

c) constructing a feature enhancement unit, wherein the feature enhancement unit is composed of one 1*1 convolution layer, an activation function layer, one 1*1 convolution layer, and one residual connection, obtaining a second output by performing series operation on a 1*1 convolution, an activation function, and an 1*1 convolution, and obtaining a third output by performing horizontal direction feature map concatenating on the original input feature map and the second output through the residual connection, that is, obtaining an output result of the multi-branch parallel convolution operation unit; and

step C) using a fully connected layer and a Sigmoid function as a combination unit to construct an n-th prediction layer, and obtaining a label recognition result of an n-th gastrointestinal endoscopic feature group through Sigmoid function operation, wherein a Sigmoid operation formula is:

$$p_k = Sigmoid(W_k^T \cdot F + b_k)$$

wherein $p_k$ indicates a probability of solving for category k, $W_k^T$ is a trainable hyperparameter for category k, and $b_k$ is a trainable biased hyperparameter for category k.

5. The system for recognizing gastrointestinal endoscopic lesion features according to claim 1 or 4, **characterized in that** a method for training the first multi-task classification neural network comprises: inputting training sets to the first multi-task classification neural network in batches for training, and specifically comprises:

step D) predicting an original label value output correspondingly by each of the gastrointestinal endoscopic feature groups, performing calculation through a BCE cross-entropy loss function, obtaining an n-th loss function, and calculating a total loss of the network in a weighted summation manner, wherein a specific formula is as follows:

$$L_k = -\frac{1}{N}\sum_{i=1}^{N}[y_i \, log(p_i) + (1-y_i) \, log(1-p_i)]$$

wherein k is a number of branches of the feature groups; $L_k$ is a derivative of a loss for k feature branches; N is a number of pictures in a training batch; i is a corresponding category; $y_i$ is a true label value of category i in the pictures, that is, $y_i$ is 1 in a case where a picture belongs to category i, and $y_i$ is 0 in a case where a picture does not belong to category i; and $p_i$ is a prediction confidence coefficient of the picture for category i;

$$L = w_1 \times L_1 + w_2 \times L_2 + \ ...... + w_k \times L_k$$

wherein L is the total loss, and w is a weight of the loss function; and the total loss is obtained by summing; and

step E) updating parameters of a neural network model by using a backpropagation technology according to the total loss of the network, stopping training in a case where a final loss function is stabilized to a preset value or reaches a preset maximum iteration number, and obtaining the first multi-task classification neural network trained in advance.

6.  The system for recognizing gastrointestinal endoscopic lesion features according to claim 3, further comprising a mucosal location recognition module configured to convert the label values in the mucosal feature group according to a mucosal location and output the recognition result to the feature result collection module;
    wherein the location comprises esophagus, gastric body, gastric fundus, gastric antrum, duodenum, cecum, ascending colon, transverse colon, descending colon, sigmoid colon, or rectum.

7.  The system for recognizing gastrointestinal endoscopic lesion features according to claim 1, **characterized in that** the local lesion features comprise at least one of a lesion morphology feature, a lesion area feature, a lesion surface feature, or a lesion border feature.

8.  The system for recognizing gastrointestinal endoscopic lesion features according to claim 1 or 7, **characterized in that** a method for constructing the second multi-task classification neural network comprises:

    step F) constructing a local lesion segmentation artificial intelligence neural network, wherein the local lesion segmentation artificial intelligence neural network is configured to classify and predict images input by the local lesion feature group, to obtain a first lesion segmentation map;
    step G) constructing a local feature multi-label recognition network based on a segmented region, wherein the local feature multi-label recognition network is configured to recognize the m preset different categories of local lesion features and complementary identification values corresponding to the categories; and
    step H) constructing a label conversion module, wherein the label conversion module is configured to fuse the complementary identification values obtained in step G) with the label value of the local lesion feature group, to obtain a new label value of the local lesion feature group.

9.  The system for recognizing gastrointestinal endoscopic lesion features according to claim 1, further comprising a disease diagnosis decision making module configured to obtain a disease diagnosis result by using the recognition results.

10. An electronic apparatus, comprising an electronic apparatus body and the system for recognizing gastrointestinal endoscopic lesion features according to any one of claims 1 to 9, wherein the system for recognizing gastrointestinal endoscopic lesion features is mounted on the electronic apparatus body.

**FIG.1**

EP 4 765 037 A1

## INTERNATIONAL SEARCH REPORT

| International application No. |
| --- |
| **PCT/CN2024/103559** |

**A.    CLASSIFICATION OF SUBJECT MATTER**

G06V 10/764(2022.01)i

According to International Patent Classification (IPC) or to both national classification and IPC

**B.    FIELDS SEARCHED**

Minimum documentation searched (classification system followed by classification symbols)

IPC: G06V, G06T, G16H

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 消化道, 内镜, 图像, 病变, 疾病, 识别, 诊断, 类别, 类型, 模型, 训练, 标签, 标注, 位置, 区域, 局部, digestive tract, scope, image, lesion, disease, identification, diagnosis, category, type, model, training, label, location, region, local

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 117036815 A (TIANJIN YUJIN ARTIFICIAL INTELLIGENCE MEDICAL TECHNOLOGY CO., LTD.) 10 November 2023 (2023-11-10)<br>claims 1-10 | 1-10 |
| X | CN 111128396 A (QILU HOSPITAL OF SHANDONG UNIVERSITY et al.) 08 May 2020 (2020-05-08)<br>description, paragraphs [0043]-[0101] | 1, 2, 5, 7, 9, 10 |
| Y | CN 111128396 A (QILU HOSPITAL OF SHANDONG UNIVERSITY et al.) 08 May 2020 (2020-05-08)<br>description, paragraphs [0043]-[0101] | 3, 6 |
| Y | CN 110993099 A (QILU HOSPITAL OF SHANDONG UNIVERSITY et al.) 10 April 2020 (2020-04-10)<br>description, paragraphs [0065]-[0089] | 3 |
| Y | US 2022189015 A1 (WANG GUOHUA) 16 June 2022 (2022-06-16)<br>description, paragraphs [0077]-[0080] | 6 |

☑ Further documents are listed in the continuation of Box C.      ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **19 September 2024** | **24 September 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/103559**

### C. DOCUMENTS CONSIDERED TO BE RELEVANT

| Category* | Citation of document, with indication, where appropriate, of the relevant passages | Relevant to claim No. |
|---|---|---|
| A | CN 110473192 A (TENCENT MEDICAL HEALTH (SHENZHEN) CO., LTD.) 19 November 2019 (2019-11-19)<br>entire document | 1-10 |

Form PCT/ISA/210 (second sheet) (July 2022)

**INTERNATIONAL SEARCH REPORT**
**Information on patent family members**

International application No.

**PCT/CN2024/103559**

| Patent document cited in search report | | | Publication date (day/month/year) | Patent family member(s) | | | Publication date (day/month/year) |
|---|---|---|---|---|---|---|---|
| CN | 117036815 | A | 10 November 2023 | None | | | |
| CN | 111128396 | A | 08 May 2020 | None | | | |
| CN | 110993099 | A | 10 April 2020 | None | | | |
| US | 2022189015 | A1 | 16 June 2022 | WO | 2021036863 | A1 | 04 March 2021 |
| | | | | JP | 2024028975 | A | 05 March 2024 |
| | | | | EP | 4018909 | A1 | 29 June 2022 |
| | | | | EP | 4018909 | A4 | 26 October 2022 |
| | | | | JP | 2022545124 | A | 25 October 2022 |
| | | | | JP | 7404509 | B2 | 25 December 2023 |
| | | | | CN | 110495847 | A | 26 November 2019 |
| CN | 110473192 | A | 19 November 2019 | WO | 2020207377 | A1 | 15 October 2020 |
| | | | | US | 2021272681 | A1 | 02 September 2021 |
| | | | | US | 11967414 | B2 | 23 April 2024 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311040222